## Europäisches Patentamt

## European Patent Office

## Office européen des brevets

(11) Veröffentlichungsnummer: **0 474 116 B1**

# EUROPÄISCHE PATENTSCHRIFT

(45) Veröffentlichungstag der Patentschrift: **26.07.95**

(21) Anmeldenummer: **91114528.2**

(22) Anmeldetag: **29.08.91**

(51) Int. Cl.⁶: **C12N 9/16**, C12Q 1/44,
//(C12N9/16,C12R1:01)

(54) Stabile 6-Phosphogluconolactonase, Verfahren zu deren Gewinnung sowie Verwendung des Enzyms.

(30) Priorität: **05.09.90 DE 4028086**

(43) Veröffentlichungstag der Anmeldung:
**11.03.92 Patentblatt 92/11**

(45) Bekanntmachung des Hinweises auf die
Patenterteilung:
**26.07.95 Patentblatt 95/30**

(84) Benannte Vertragsstaaten:
**AT BE CH DE DK ES FR GB GR IT LI LU NL SE**

(56) Entgegenhaltungen:
EP-A- 0 043 181
EP-A- 0 328 380
US-A- 3 907 644

**BRITISH JOURNAL OF HAEMATOLOGY vol.
62, no. 3, 1986, OXFORD GB Seiten 577 -586;
Beutler E. et al.: "Blood cell phosphogluconolactonase assay and properties**

**EMBASE DATABASE,Excerpta Medica,Amsterdam,NL, Abstract
No.86216511,Beutler E.et al.: "Characteristics
and significance of the reverse glucose-
6-phosphatedehydrogenase reaction" &**

J.Lab.Clin. Med.,1986,107/6,502-7

(73) Patentinhaber: **BOEHRINGER MANNHEIM
GMBH**

**D-68298 Mannheim (DE)**

(72) Erfinder: **Moellering, Hans
Herrestrasse 10
W-8132 Tutzing (DE)**
Erfinder: **Kresse, Georg-Burkhard, Prof. Dr.
Alpspitzstrasse 6
W-8122 Penzberg (DE)**

**Beschreibung**

Die Erfindung betrifft eine stabile 6-Phosphogluconolactonase, EC 3.1.1.31, ein Verfahren zur Gewinnung des Enzyms sowie dessen Verwendung zur Bestimmung klinisch relevanter Parameter, die sich in 6-Phosphogluconolacton überführen lassen bzw. an dessen Bildung direkt oder indirekt beteiligt sind.

Insbesondere betrifft die Erfindung eine 6-Phosphogluconolactonase (PGL) mit guter Stabilität, welche aus Mikroorganismen wie Leuconostoc-Bakterien gewonnen werden kann.

PGL - der systematische Name lautet 6-Phospho-D-glucono-1,5-lactone lactonohydrolase - katalysiert die Hydrolyse von 6-Phosphogluconolacton zu 6-Phosphogluconat. 6-Phosphogluconolactonase kann daher für die Bestimmung von 6-Phosphogluconolacton und von Reaktionspartnern, die in 6-Phosphogluconolacton direkt oder über mehrere Reaktionsstufen überführbar sind bzw. von Enzymen, die entsprechende Reaktionen katalysieren, verwendet werden. Insbesondere ist der PGL-Weg für die Bestimmung von Glucose, ATP und ATP-bildenen Reaktionspartnern geeignet. Durch den Zusatz von 6-Phosphogluconat-Dehydrogenase werden so zwei Reduktionsäquivalente aus einem Mol ATP erhalten. Entsprechende Bestimmungen können somit mit doppelter Empfindlichkeit durchgeführt werden. Dieses Prinzip ist in der Literatur beschrieben (Vormbrock und Helger, Enzyme $\underline{38}$, Suppl. 1, 20 - 21, 1987).

Es ist bekannt, daß PGL in Hefen sowie verschiedenen eukaryotischen Organen wie Leber, Niere, Erythrocyten, Gewebe und in einigen Mikroorganismen vorkommt.

PGL wurde erstmals in Hefeextrakten entdeckt (Brodie und Lipman, J. Biol. Chem. $\underline{212}$, 677 - 685, 1955). Kawada et al. reinigten PGL aus Hefe, wobei eine über 130fache Anreicherung erzielt werden konnte (Biochem. Biophys. Acta $\underline{57}$, 404 - 407, 1962). Dieses Enzympräparat verfügte jedoch nicht über die für quantitative Bestimmungen erforderliche Substratspezifität. Ein homogenes Enzympräparat mit in dieser Hinsicht verbesserten Eigenschaften konnte aus Rinder-Erythrozyten gewonnen werden (Bauer et al., Eur. J. Biochem. $\underline{133}$, 163 - 168, 1983). Des weiteren ist ein teilweise aufgereinigtes PGL-Enzym aus menschlichen Blutzellen beschrieben (Beutler, E. et al., Brit. J. Haemat. $\underline{62}$, 577 - 586, 1986). Die am besten untersuchte Phosphogluconolactonase aus Mikroorganismen ist das aus dem Bakterium Zymomonas mobilis (Scopes, FEBS Lett. $\underline{193}$, (2) 185 - 188, 1985). Danach ist PGL in Zymomonas mobilis in großen Mengen enthalten und mit einer ca. 500fachen Anreicherung zu gewinnen. Zudem zeigt das Zymomonas-Enzym eine gute Substratspezifität ($K_m$ 0,02 - 0,03 mmol/l).

Nachteilig ist jedoch bei allen bisher bekannten PGL-Enzymen, daß die Stabilität der isolierten Enzyme unzureichend ist, und daß die Gewinnung der Enzyme aus tierischen Organen und den beschriebenen Mikroorganismen sehr aufwendig und zeitraubend ist. Darüber hinaus stehen die benötigten Quellen in den meisten Fällen nicht in ausreichender Menge zur Verfügung bzw. kommen nur an wenigen, speziellen Plätzen vor. Die beschriebenen PGL-Enzyme aus Hefe verfügen darüber hinaus nicht über die erforderliche Substratspezifität.

Der Erfindung liegt daher die Aufgabe zugrunde, eine stabile 6-Phoshpogluconolactonase zur Verfügung zu stellen, die darüber hinaus in leicht kultivierbaren, anspruchslosen, ubiquitär vorkommenden Mikroorganismen aufzufinden ist.

Diese Aufgabe wird gelöst, indem eine 6-Phosphogluconolactonase zur Verfügung gestellt wird, die nach ungefähr 20 Stunden bei pH 7,0 und 20 °C noch wenigstens 90 % PGL-Restaktivität aufweist, und aus einem Leuconostoc-Stamm erhältlich ist. In der Regel zeigt das Enzym nach ungefähr 20 Stunden unter den genannten Bedingungen sogar noch die volle PGL-Ausgangsaktivität. Überhaupt weist die aus Leuconostoc erhältliche Phosphogluconolactonase eine gute Aktivität zwischen 20 und 30 °C und bei einem pH-Wert von 5,8 bis 9,0 auf. Der optimale pH-Wert liegt bei 6,5 bis 7,0; die optimale Temperatur gleichbleibend zwischen 20 und 30 °C. Die katalytische Effizienz der PGL für 6-Phosphogluconolacton entspricht mit einer Michaelis-Konstante, $K_m$, kleiner als 0.1 mmol/l der für quantitative Bestimmungen erforderlichen Qualität.

Ein weiterer Gegenstand der Erfindung ist ein Verfahren zur Gewinnung von 6-Phosphogluconolactonase aus Mikroorganismen durch Züchtung derselben und Gewinnung des Enzyms aus der Biomasse oder der Kulturbrühe, dadurch gekennzeichnet, daß man einen Leuconostoc-Stamm züchtet. Bevorzugt werden Leuconostoc mesenteroides und besonders bevorzugt die öffentlich zugängliche Leuconostoc mesenteroides-Subspezies dextranicum (DSM 20187, NCIB 3355) verwendet.

Die erfindungsgemäßen Leuconostoc-Stämme lassen sich höchst einfach auf einem Glucose (oder auch Lactose) als Kohlenstoffquelle und Hefe-Extrakt als Stickstoffquelle sowie Salze und Spurenelemente enthaltenden Medium züchten.

Als geeignete Salze können u. a. $MgSO_4$ und $K_2HPO_4$ eingesetzt werden. Geeignete Spurenelemente sind u. a. Mn und Fe.

Besonders hohe Ausbeuten an PGL lassen sich erzielen, wenn man kontinuierlich Substrat bei 10 - 37 °C und geregeltem pH-Wert von ungefähr 5.2 - 7.2 zufüttert.

2

Die Isolierung des Enzyms kann nach den üblichen Methoden der Enzymreinigung erfolgen, beispielsweise wie in Eur. J. Biochem. 133, 163 ff (1983) und in FEBS Lett. 193, 185 ff. (1985) beschrieben. Bevorzugt wird jedoch ein Anreicherungsverfahren, bei dem nach Aufschluß, beispielsweise durch Lysozym-Zusatz, eine Polimin-Behandlung und anschließend eine fraktionierte Ammoniumsulfat-Fällung durchgeführt wird und die $(NH_4)_2 SO_4$-Fraktion (ca. 1,4 - 3,2 M) über Phenylsepharose chromatographiert wird. Anschließend erfolgt eine Gelfiltration, beispielsweise an Sephacryl S200 oder Superdex 200. Nach diesem Verfahren wird ein PGL-Präparat mit einer Ausbeute von über 50 % mit einer spezifischen Aktivität von mindestens 500 U/mg Protein erhalten. Einzelne Enzym-Präparate zeigen eine spezifische Aktivität von ungefähr 1500 U/mg Protein.

Das auf diese Weise gereinigte Enzym kann direkt für analytische Zwecke eingesetzt werden. PGL ist technisch insbesondere von Interesse im Rahmen der Bestimmungen von Glucose-6-phospat, Glucose, ATP und ATP-bildenen Enzymreaktionen, wie z. B. die enzymatische Bestimmung von Creatin-Kinase oder Creatinin bzw. Creatin. Alle Bestimmungsmethoden, bei denen 6-Phosphogluconolacton gebildet werden, können durch die PGL-Katalyse beschleunigt werden, da 6-Phosphogluconolacton chemisch nur langsam zur Säure umgelagert wird. Die demgemäß zugesetzte PGL und nachgeschaltete 6-Phosphogluconat-Dehydrogenase (6-PGDH) resultiert in einer Erhöhung, im Idealfall in einer Verdopplung der Empfindlichkeit nach sehr viel kürzerer Zeit. Somit sind solche gekoppelten Teste besonders für Parameter mit kleinem Meßsignal von Bedeutung. Dies trifft beispielsweise für CK-MB-Isoenzym-Messungen im Serum oder Blut von Herzinfarkt-Patienten zu (Vormbrock und Helger, s.o.). Neben den erforderlichen Hilfsenzymen wie z. B. Hexokinase (HK), Glucose-6-phosphat-Dehydrogenase (G6PDH), PGL und 6-PGDH wird außerdem $NAD^+$ bzw. $NADP^+$ als Coenzym zugesetzt. Welches der beiden Coenzyme eingesetzt wird, hängt lediglich von der Quelle der verwendeten Hilfsenzyme G6PDH und 6-PGDH ab, ist jedoch für die eingesetzte PGL aus Leuconostoc ohne Bedeutung. Somit entstehen aus 1 Mol ATP 2 Mol NADH bzw. 2 Mol NADPH, $CO_2$ und Ribulose-5-phosphat als Endprodukte der Enzymkaskade. Das gebildete NADH bzw. NADPH wird bei einer Wellenlänge von 340 oder 365 nm in Reflexionsphotometer detektiert. Die erfindungsgemäße PGL aus Leuconostoc wird in einer Menge von 0,2 - 0,5 U/ml Testlösung, vorzugsweise 0,3 U/Test-ml zugesetzt.

Besondere Bedeutung kommt dem Zusatz von PGL zur Beschleunigung entsprechender Bestimmungen zu, wenn eine G6PDH verwendet wird, die keine die PGL-Reaktion beeinflussende Komponenten enthält, was beispielsweise bei rekombinant hergestellter G6PDH der Fall ist. Dadurch wird der Zusatz einer definierten Menge an PGL möglich, die Beschleunigung der Reaktion und somit die Verdoppelung des Meßsignals kalkulierbarer und demzufolge entsprechende Teste reproduzierbarer.

Ein geeignetes diagnostisches Mittel enthält die erfindungsgemäße PGL und die oben erwähnten Hilfsenzyme bzw. Coenzyme. Sämtliche Inhaltsstoffe können vereinigt ungefähr 24 Stunden bei ca. 20 bis 30°C gelagert werden und kurz vor Ausführung der Bestimmung direkt mit der zu untersuchenden Probe vermischt werden.

Erläuterungen zu den Abbildungen:

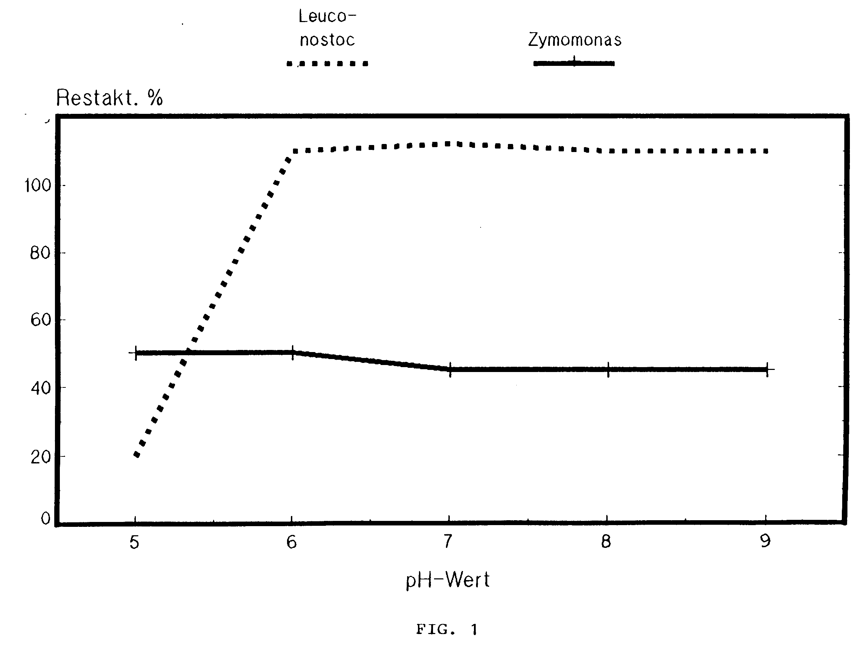

Figur 1    pH-Stabilität: Restaktivität in % bei zunehmendem pH-Wert bei + 20°C für PGL aus Leuconostoc und PGL aus Zymomonas (0,2 U/ml PGL in 50 mmol/l Kaliumphosphat-Puffer; 2 Stdn.)

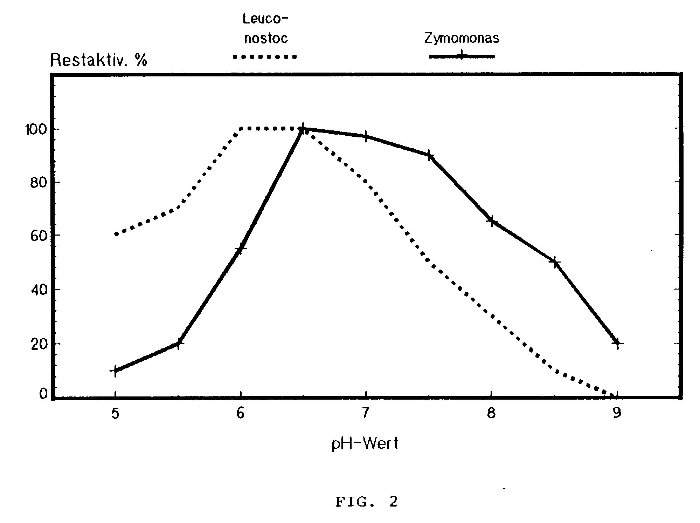

Figur 2    Aktivitätsoptimum: Restaktivität in % bei zunehmendem pH-Wert bei + 25°C für PGL aus Leuconostoc und PGL aus Zymomonas (0,1 M MES, 2 mmol/l $MgCl_2$, 30 mmol/l NaCl) MES = 4-Morpholinethansulfonsäure

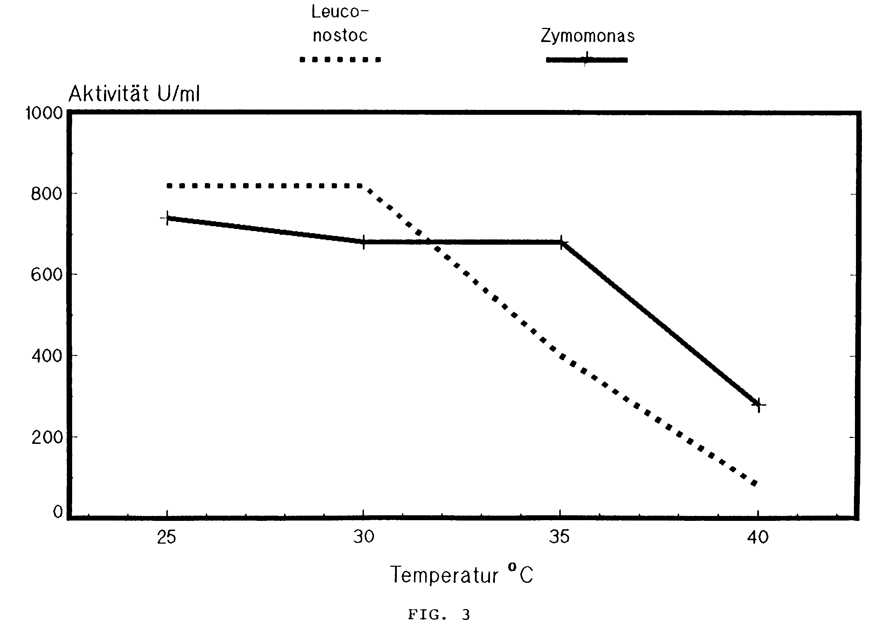

Figur 3    Temperaturabhängigkeit: Aktivität in U/ml mit zunehmender Temperatur bei pH 6,5 für PGL aus Leuconostoc und PGL aus Zymomonas.

Die folgenden Beispiele erläutern die Erfindung weiter:

Beispiel 1

Fermentation von Leuconostoc:

In einem 100 l-Fermenter wurde ein Nährmedium dampfsterilisiert, welches als Hauptkomponenten Hefe-Extrakt, Glucose, Salze und Spurenelemente enthielt. Der 100 l-Fermenter wurde mit einer 3 l-Kultur von Leuconostoc mesenteroides sp. dextranicum DSM 20187 beimpft, welche zuvor bis zu einer optischen Dichte von 3 gezüchtet worden war.

Die 100 l-Kultur wurde mit einer Belüftungsrate von 0,2 vvm mit Luft versorgt und die Temperatur bei 25° C konstant gehalten, während des gesamten Prozesses wurde der pH-Wert der Kultur mit Natronlauge bei pH 6,8 geregelt. Hierbei reichert sich mit zunehmender Biomasse die PGL in den Zellen an. Nach 16 h wurden die Bakterien über eine Padberg-Zentrifuge geerntet und aus den Zellen die PGL isoliert.

Beispiel 2

Isolierung von 6-Phosphogluconolactonase (PGL) aus Leuconostoc-Fermentationsbrühe:

100 Liter Fermentations-Kultur Leuconostoc dextranicum wurden zentrifugiert. Es resultieren 1470 g Zellen-Feuchtmasse, entsprechend 320 g Trockenmasse. Die Biomasse wurde mit 15 Liter 10 mmol/l Kaliumphosphat-Puffer, pH 6.5, suspendiert, mit 7,35 g Lysozym versetzt und 12 Stunden bei 20°C aufgeschlossen. Zur vollständigen Lyse wurde 2mal bei 700 bar unter Eiskühlung eine Hochdruck-Dispersion vorgenommen. Zur Abtrennung der Nucleinsäuren und Zelltrümmer wurde eine Behandlung mit 1,2 % Polimin G 20 (BASF) durchgeführt. Das Enzym verbleibt nach Zentrifugation oder Filtration vollständig im Überstand. Anschließend wurde zur Enzymlösung DEAE-Sephadex, äquilibriert mit 20 mmol/l Kaliumphosphat-Puffer, pH 6.5, zugeführt und 60 min bei + 4°C gerührt. Der Anionenaustauscher wurde von der Lösung abgetrennt und 2mal mit je 1 L 100 mmol/l Kaliumphosphat-Puffer enthaltend 0,8 mol/l Ammoniumsulfat, pH 6.5, eluiert. Die vereinigten Eluate wurden mit festem Ammoniumsulfat zwischen 1,4 und 3,2 mol/l fraktioniert. Der präzipitierte PGL-Niederschlag wurde über Phenylsepharose mittels eines absteigenden Gradienten (1,5 mol/l Ammoniumsulfat, 20 mmol/l Kaliumphosphat, pH 6.5) chromatographiert. Das Enzym kommt bei ca. 1 mol/l Ammoniumsulfat von der Säule. Nach Ankonzentrieren der aktiven Fraktionen auf 50 mg Protein/ml bei ungefähr + 4°C wurde, nach anschließender Molekularsieb-Passage an einer Sephacryl S200-Säule, ein PGL-Präparat mit über 500 U/mg Protein erhalten. Abhängig vom verwendeten Säulenmaterial können PGL-Fraktionen mit einer spezifischen Aktivität von ungefähr 1500 U/mg erreicht werden.

Die Fremdaktivitäten NADH-Oxidase, G6PDH, 6PGDH, ATPase, Hexokinase, Creatin-Kinase, Phosphoglucomutase, Glutathion-Reduktase, Phosphoglucose-Isomerase, Glucose-Dehydrogenase und Glucose-Oxidase sind alle kleiner als 0,01 %, bezogen auf die PGL-Aktivität.

Das Enzym wird mit Ammoniumsulfat auf 3,2 mol/l gesättigt und bei 10 mg Protein/ml, pH 6.5, + 4°C aufbewahrt und in dieser Form zur analytischen Bestimmung eingesetzt.

Tabelle 1

| Reinigung von PGL aus Leuconostoc (100 L-Kultur) | | | | |
|---|---|---|---|---|
| Schritt | KU | U/mg | Protein (g) | Ausbeute [in %] |
| Lysozym + Hochdruck | 1890 | 10 | 189 | 100 |
| $G_{20}$-Überstand | 1500 | 44 | 34 | 79 |
| DEAE-Eluate | 1640 | 54 | 30 | 86 |
| AS-Fraktion 1,4-3,2 M | 1600 | 64 | 25 | 84 |
| Phenyl-Sepharose-Eluate | 1250 | 290 | 4.3 | 66 |
| Mol-Sieb-Passage an Sephacryl S200 | 1050 | 590 | 1.8 | 55 |

Beispiel 3

Stablität von PGL aus Leuconostoc

Eine nach Beispiel 2 erhältliche PGL-Fraktion sowie das von Scopes aus Zymomonas gewonnene Enzym werden in einer Konzentration von jeweils 0,2 U/ml in 50 mmol/l Kaliumphosphat, pH 7.0, bei 20°C gehalten. Nach 2 bzw. 20 Stunden wurde die verbliebene Enzymaktivität bestimmt (s. Tab. 2).

Tabelle 2

| Restaktivität in % nach 20 Stunden | | |
|---|---|---|
| | Restaktivität % | |
| | 2 Std. | 20 Std. |
| Zymomonas mob. | 45 | 0 |
| Leuconostoc dex. | 100 | 100 |

Beispiel 4

Bestimmung von Creatin-Kinase

I. Lösungen

1. Puffer: Imidazol (0.1 mol/l; pH 6.7), Glucose(20 mmol/l), Mg-Acetat (10 mmol/l), EDTA (2 mmol/l)
2. Creatinphosphat-Lösung (30 mmol/l)
3. ADP-Lösung (2 mmol/l)
4. Hexokinase-Lösung (2.5 U/ml)
5. NAD- bzw. NADP-Lösung (2 mmol/l)
6. Glucose-6-Phosphat-Dehydrogenase-Lösung (1.5 U/ml)
7. PGL-Lösung (1 U/ml)
8. 6-Phosphogluconat-Dehydrogenase (1 U/ml)
Verdünnungen der Probe in Imidazol-Puffer.

II. Ausführung

Meßstrahlung 365 nm; Schichtdicke 1,0 cm; Testvolumen 0.5 ml; Temperatur 37 °C; Extinktionskoeffizient $\epsilon$ = 3.4 bzw. 3,5 $cm^2/\mu mol$. Probevolumen 0.02 ml;

III. Berechnung

$$\text{Vol.-Aktivität} = \frac{\Delta E/min \times 0,5 \ ml \times \text{Verdünnung}}{\times \ \text{eingesetztes Vol. [ml]}} = U/ml \ \text{Probe}$$

IV. Ergebnis

Linearität, prozentuale Standardabweichung und daher Präzision sind im Vergleich mit Standardmethode, z. B. mit "CK NAC aktiviert" von Boehringer Mannheim, vergleichbar bzw. besser (s. Tabelle 3).

Tabelle 3

| Vergleichende Bestimmung von CK-MB nach herkömmlicher Methode (1) und mit Zusatz von PGL (Methode 2) | | | | |
|---|---|---|---|---|
| | | | HS 1 | HS 2 |
| Methode 1 | | $\overline{x}$ | 24,7 | 15,8 |
| | | S | 1,8 | 2,1 |
| | | $V_K$ | 7,5 | 13,2 |
| | | n | 19 | 18 |
| Methode 2 | | $\overline{x}$ | 48,9 | 29,1 |
| | | S | 2,1 | 1,2 |
| | | $V_K$ | 4,2 | 4,3 |
| | | n | 19 | 18 |
| HS 1, HS 2: 2 verschiedene, nicht-pathogene Humanseren; $\overline{x}$ = Mittelwerte in U/l CK-MB-Aktivität; S = Standardabweichung in U/l; $V_K$ = prozentuale Standardabweichung; n = Anzahl der Messungen; Methode 1: "CK NAC aktiviert", Boehringer Mannheim GmbH; Methode 2: dito + 1U/ml 6-PGDH + 1 U/ml PGL. | | | | |

Insbesondere bei niedrigen Konzentrationen an CK-MB werden oft Impräzision beobachtet. Die CK-MB-Bestimmung wird durch entsprechenden Enzymzusatz und dadurch bewirkte Verdopplung des NAD(P)H-Meßsignals verbessert. Dies kommt durch wesentlich niedrigere $V_K$-Werte bei Methode 2 gegenüber Methode 1 zum Ausdruck. Die Verdopplung des Meßsignals spiegeln die entsprechenden x-Werte bei identischem Berechnungsfaktor wider. Für die Berechnung der CK-MB-Konzentration ist daher zusätzlich der Tatsache Rechnung zu tragen, daß pro Mol Creatinphosphat zwei Mole NADH bzw. NADPH gebildet werden.

Entsprechend können andere Parameter, wie z. B. ATP und Glucose bestimmt werden.

**Patentansprüche**

1.  6-Phosphogluconolactonase (PGL) erhältlich aus Mikroorganismen der Gattung Leuconostoc, dadurch gekennzeichnet, daß nach ungefähr 20 Stunden bei pH 7,0 und 20°C noch wenigstens eine PGL-Aktivität von 90% vorhanden ist.

2.  6-Phosphogluconolactonase nach Anspruch 1, dadurch gekennzeichnet, daß das Enzym aus Leuconostoc mesenteroides erhältlich ist.

3.  6-Phosphogluconolactonase nach Anspruch 2, dadurch gekennzeichnet, daß das Enzym aus der Subspezies dextranicum (DSM 20187, NCIB 3385) erhältlich ist.

4.  6-Phosphogluconolactonase nach einem der Ansprüche 1 bis 3, dadurch gekennzeichnet, daß das Enzym die folgenden Merkmale aufweist:
    (1) optimaler pH-Wert 6,5 - 7,0;
    (2) optimale Temperatur von ungefähr 25°C; und
    (3) Michaelis-Konstante kleiner als 0,1 mmol/l.

5.  Verfahren zur Gewinnung von 6-Phosphogluconolactonase nach einem der Ansprüche 1 bis 4 aus Mikroorganismen durch Züchtung derselben und Gewinnung des Enzyms aus der Biomasse oder der Kulturbrühe, dadurch gekennzeichnet, daß man einen Leuconostoc-Stamm züchtet.

6.  Verfahren nach Anspruch 5, dadurch gekennzeichnet, daß man Leuconostoc mesenteroides dextranicum (DSM 20187, NCID 3355) züchtet.

7.  Verfahren nach Anspruch 5 oder 6, dadurch gekennzeichnet, daß der Mikroorganismus unter kontinuierlicher Substratzufütterung bei 10 bis 37°C und geregeltem pH-Wert von ungefähr 5,2 - 7,2 gezüchtet wird.

8. Verwendung einer 6-Phosphogluconolactonase nach einem der Ansprüche 1 bis 4 zur Bestimmung von 6-Phosphogluconolacton und Reaktionspartnern, die enzymatisch in 6-Phosphogluconolacton überführbar sind bzw. an diesen Reaktionen beteiligten Enzymen.

9. Verwendung nach Anspruch 8, dadurch gekennzeichnet, daß Creatin-Kinase, Creatinin, Creatin, Glucose oder ATP bestimmt wird.

10. Diagnostisches Mittel enthaltend eine 6-Phosphogluconolactonase nach einem der Ansprüche 1 bis 4, dadurch gekennzeichnet, daß das Mittel die Hilfsenzyme Hexokinase, G6PDH und 6-PGDH sowie die Coenzyme NAD$^+$ und/oder NADP$^+$ sowie die erforderlichen Salze und Puffersubstanzen enthält.

**Claims**

1. 6-Phosphogluconolactonase (PGL) obtained from micro-organisms of the genus Leuconostoc, characterised in that, after about 20 hours at pH 7.0 and 20°C, a PGL activity of at least 90% is still present.

2. 6-Phosphonogluconolactonase according to claim 1, characterised in that the enzyme is obtainable from Leuconostoc mesenteroides.

3. 6-Phosphonogluconolactonase according to claim 2, characterised in that the enzyme is obtainable from the subspecies dextranicum (DSM 20187, NCIB 3385).

4. 6-Phosphonogluconolactonase according to one of claims 1 to 3, characterised in that the enzyme displays the following characteristics:
   (1) optimum pH value 6.5 - 7.0;
   (2) optimum temperature of about 25°C; and
   (3) Michaelis constant smaller than 0.1 mmol/l.

5. Process for the obtaining of 6-phosphonogluconolactonase according to one of claims 1 to 4 from micro-organisms by culturing thereof and obtaining of the enzyme from the biomass or from the culture broth, characterised in that one cultures a Leuconostoc strain.

6. Process according to claim 5, characterised in that one cultures Leuconostoc mesenteroides (DSM 20187, NCID 3355).

7. Process according to claim 5 or 6, characterised in that the micro-organism is cultured with continuous substrate feed addition at 10 to 37°C and regulated pH value of about 5.2 - 7.2.

8. Use of a 6-phosphonogluconolactonase according to one of claims 1 to 4 for the determination of 6-phosphonogluconolactone and of reaction partners which are convertable enzymatically into 6-phosphogluconolactone or of enzymes participating in these reactions.

9. Use according to claim 8, characterised in that creatine kinase, creatinine, creatine, glucose or ATP is determined.

10. Diagnostic agent containing a 6-phosphonogluconolactonase according to one of claims 1 to 4, characterised in that the agent contains auxiliary enzymes hexokinase, G6PDH and 6-PGDH, as well as the co-enzymes NAD$^+$ and/or NADP$^+$, as well as the necessary salts and buffer substances.

**Revendications**

1. 6-Phosphogluconolactonase (PGL) pouvant être obtenue à partir de micro-organismes du genre Leuconostoc, caractérisée en ce que, après environ 20 heures à pH 7,0 et à 20°C, on trouve encore une acivité de PGL d'au moins 90 %.

2. 6-Phosphogluconolactonase selon la revendication 1, caractérisée en ce que l'enzyme peut être obtenue à partir de Leuconostoc mesenteroides.

**3.** 6-Phosphogluconolactonase selon la revendication 2, caractérisée en ce que l'enzyme peut être obtenue à partir de la sous-espèce dextranicum (DSM 20187, NCIB 3385).

**4.** 6-Phosphogluconolactonase selon l'une quelconque des revendications 1 à 3, caractérisée en ce que l'enzyme présente les caractéristiques suivantes :
   (1) un pH optimal de 6,5-7,0 ;
   (2) une température optimale d'environ 25 °C ; et
   (3) une constante de Michaelis inférieure à 0,1 mmole/l.

**5.** Procédé de préparation de 6-phosphogluconolactonase selon l'une quelconque des revendications 1 à 4, à partir de micro-organismes, par culture de ces derniers et récupération de l'enzyme à partir de la biomasse ou du milieu de culture, caractérisé en ce que l'on cultive une souche de Leuconostoc.

**6.** Procédé selon la revendication 5, caractérisé en ce que l'on cultive Leuconostoc mesenteroides dextranicum (DSM 20187, NCID 3355).

**7.** Procédé selon la revendication 5 ou 6, caractérisé en ce que l'on cultive les micro-organismes tout en alimentant constamment du substrat à une température de 10 à 37 °C et à un pH ajusté à environ 5,2-7,2.

**8.** Utilisation d'une 6-phosphogluconolactonase selon l'une quelconque des revendications 1 à 4, pour la détermination de 6-phosphogluconolactone et de partenaires réactionnels, qui peuvent être transformés par voie enzymatique en 6-phospnogluconolactone ou en enzymes participant à ces réactions.

**9.** Utilisation selon la revendication 8, caractérisée en ce que l'on détermine la créatinekinase, la créatinine, la créatine, le glucose ou l'ATP.

**10.** Agent de diagnostic contenant une 6-phosphogluconolactonase selon l'une quelconque des revendications 1 à 4, caractérisé en ce que l'agent contient les coenzymes hexokinase, G6PDH et 6-PGDH ainsi que les coenzymes $NAD^+$ et/ou $NADP^+$ ainsi que les sels et tampons nécessaires.

FIG. 1

Restaktiv. %

Leuconostoc

Zymomonas

100
80
60
40
20
0

5   6   7   8   9

pH−Wert

FIG. 2

FIG. 3

EP 0 474 116 B1